# EUROPEAN PATENT APPLICATION

(11) **EP 0 787 481 A1**
(43) Date of publication of application: **06.08.1997**
(21) Application number: 97300429.4
(22) Date of filing: 23.01.1997
(51) Int. Cl.: A61K 7/30

(54) **Denture cleansing compositions and tablets**

(30) Priority: 30.01.1996 GB 9601784
(71) Applicant: Kukident GmbH, D-69469 Weinheim (DE)
(72) Inventor: Knollmann, Rainer, 32052 Herford (DE); van de Loecht-Blasberg, Monika, 69469 Weinheim (DE); Rosentengel, Michael, 67354 Romerberg (DE)
(74) Representative: Dale, Martin Nicholas

(57) **Abstract**

A composition for use in denture cleansing comprises
a) particles containing at least one plaque and/or stain removing and/or preventing enzyme, and
b) a water-soluble or water-dispersable matrix,
with the particles being dispersed in the matrix and being visible discrete entities and said composition further comprising at least one inorganic persalt bleaching agent. The composition provides excellent cleansing and anti-bacterial activity in combination with improved manufacturing characteristics.

## Description

The invention relates to a composition for use in denture cleansing and to denture cleansing tablets made therefrom.

It is sometimes necessary for humans to replace teeth which have been lost. Artificial teeth, which are implanted into the remaining jawbone, can be treated like ordinary teeth from the point of view of hygiene. In contrast, dentures must be specially treated to remove the accumulation of plaque and mucilageneous and bacterial deposits which collect while the denture is being worn. This is also the case for braces being worn by many children nowadays.

Denture cleanser products such as effervescence tablets and powders are well known in the art.

Denture cleansing tablets are known which consist of two phases. The first phase of such tablets is responsible for conditioning the medium and providing an oxygen-free cleansing procedure step. The second phase is responsible for a second cleansing step making use of oxygen, thereby oxidizing the primary and secondary plaque already attacked in the first step of the cleansing procedure mediated by the first phase of the denture cleansing tablet.

Typically, two-phase cleansing systems are generated by two-layer tablets or the like, which impose tremendous problems to the manufacturer, as during the manufacturing process of such denture cleansing tablets, a first layer has to be created which is hard enough to survive as a pre-tablet and yet soft enough to allow key-binding of the following second layer which is pressed on the first one. To fulfill these special requirements, finely balanced formulations and respective pressure are needed, which is disadvantageous with regard to cost and environmental performance.

We have now devised a composition for use in dental cleansing that reduces or overcomes the disadvantages of known dental cleansing compositions.

In particular, the present invention provides a composition for use in denture cleansing that shows superior cleansing properties to the known compositions and improved manufacturing characteristics.

According to one aspect of the invention, there is provided a composition for use in denture cleansing comprising
a) particles containing at least one plaque and/or stain removing and/or preventing enzyme, and
b) a water-soluble or water-dispersable matrix , with the particles being dispersed in the matrix and being visible discrete entities and said composition further comprising at least one inorganic persalt bleaching agent.

The invention also provides an improved denture cleansing tablet that contains a composition according to the present invention.

Furthermore, the invention provides the use of the composition of the invention in a denture cleansing process.

The invention further provides the use of the composition of the invention to remove plaque and/or stains from teeth and/or to prevent plaque and/or stains from forming on teeth.

The invention further provides the use of the composition of the invention in the manufacture of a medicament to remove plaque and/or stains from teeth and/or to prevent plaque and/or stains from forming on teeth.

Preferably, the enzyme is a protease, lipase or glycosidase. Suitable proteases are pepsin, papain, trypsin, acidic proteases, and alkali proteases, for example. A suitable glycosidase is a glucanase, for example, an endoglucanase or exoglucanase. A suitable endoglucanase is an amylase.

Preferably, the enzyme is produced by genetic engineering techniques. Alternatively, the enzyme may be isolated from biological material originally producing said enzymatic activity.

In an embodiment of the present invention, the particles contain a mixture of at least two enzymatic activities. The particles may also contain further proteinaceous material.

The enzyme may be of animal origin and/or plant origin and/or microbial origin.

In a further embodiment of the present invention, the inorganic persalt bleaching agent is an alkali metal persulphate, an alkali metal perborate, an alkaline earth metal perborate or a mixture of two or more thereof.

The composition may also contain percarbonate as a further inorganic persalt bleaching agent. Alternatively, the composition can comprise a percarbonate as the sole inorganic persalt bleaching agent.

The or each or any combination of the inorganic persalt bleaching agent(s) may be used and/or be present as an additional particulate fraction and/or may form part of any other particulate fraction of the composition.

In accordance with the present invention, the or each or any combination of the inorganic persalt bleaching agent(s) may be present as visible discrete entities and/or may form part of any other visible discrete entities of the composition.

In an embodiment of the present invention, said composition further comprises at least one organic peroxyacid bleach precursor. The or each organic peroxyacid bleach precursor may form part of the particles containing at least one plaque and/or stain removing and/or preventing enzyme. The or each organic peroxyacid bleach precursor may also be present in the matrix.

In a preferred embodiment of the present invention, at least one organic peroxyacid bleach precursor is used and is present as an additional particulate fraction.

A most preferred embodiment of the invention is characterized by the organic peroxyacid bleach precursor being visible discrete entities.

Preferably, the composition of the invention contains at least one organic peroxyacid bleach precursor in the particles containing at least one plaque and/or stain removing and/or preventing enzyme, and/or in the matrix and/or forms an additional particulate fraction and/or forms visible discrete entities.

Preferably, the particles and/or particulate fraction and/or visible discrete entities of the composition of the invention comprise an effervescence generator.

In an embodiment of the present invention, the organic peroxyacid bleach precursor is selected from acylated polyalkyldiamines, especially tetraacetylethylenediamine, and carboxylic esters having the general formula AcL wherein Ac is the acyl moiety or an organic carboxylic acid comprising an optionally substituted, linear or branched C₆-C₂₀ alkyl or alkenyl moiety or a C₆-C₂₀ alkyl-substituted aryl moiety and L is a leaving group, the conjugate acid of which has a pKa in the range from 4 to 13.

Preferably, the water-soluble or water-dispersable matrix comprises a solid base material. In a preferred embodiment of the present invention, the solid base material releases carbon dioxide and/or oxygen with effervescence.

It is preferred that the inventive composition is characterized by the inorganic persalt bleaching agent being selected from the group comprising alkali metal perborate and/or alkaline earth metal perborate and/or percarbonate, and the solid base material comprising at least one component phase having an alkaline, an acid or neutral pH in aqueous medium, said phase having incorporated therein at least a portion of the alkali metal perborate and/or the alkaline earth metal perborate and/or the percarbonate.

The organic peroxyacid bleach precursor containing particles, particulate fraction or visible discrete entities as defined herein may have an alkaline, acidic or neutral pH in aqueous medium.

In accordance with the present invention, the solid base material may comprise a (bi)carbonate/acid effervescent couple, wherein at least the acid or neutral phase incorporates both components of the effervescent couple.

In an embodiment, the inventive composition further comprises a flavouring agent, an agent for plaque removal, tartar removal, plaque control, tartar control, surface modification, natural teeth preservation, saliva regulation, bad breath neutralization, freshness, an anti-soil/anti-bacterial agent or a mixture of two or more thereof.

In a preferred embodiment of the inventive composition at least one of said agents or any combination of said agents is used and/or is present as an additional particulate fraction or visible discrete entities.

In another preferred embodiment of the present invention at least one of said agents or any combination of said agents forms part of any particulate fraction of the composition and/or any visible discrete entities contained therein. Alternatively, at least one of said agents or any combination of said agents is present in the matrix.

The inventive composition for use in denture cleansing offers many advantages compared to those compositions known in the state of the art.

For example, as the inventive composition comprises particles containing at least one plaque and/or stain removing and/or preventing enzyme, with these particles being dispersed in a matrix and being visible discrete entities, the effect of the enzyme particles on the denture cleansing process can be visualized.

Another advantage of the inventive composition which arises from the enzyme particles being visible discrete entities is that it is possible to monitor the correct distribution of the particles in the matrix. This is of great importance for the manufacturing process. Because of the consistently high quality which can thus be guaranteed, the efficacy of a denture cleansing process which makes use of said inventive composition is increased.

A further advantage of the fact that the enzyme particles are visible discrete entities dispersed in the matrix, is that it is possible to mask any undesirable colour of the enzymes per se. Such undesirable colour of the enzymes or of the respective enzyme preparations might arise from coenzymes or any contaminants. This is of importance because of aesthetic considerations.

Additionally, the use of particles dispersed in a matrix allows the controlled use of the enzyme enclosed or associated with the particle at the level of the individual particle rather than at the level of a complete layer incorporating the enzymatic activity.

The releasing kinetics of the enzyme containing particles from the matrix they are dispersed in, as well as the releasing kinetics of the enzymes from the particles themselves can be realized in many different ways, such as, for example, by diverse coatings, use of salts, such as sodium chloride and sodium sulphate, or compounds which are rapidly or slowly soluble or by using fillers or polymers such as proteins, cellulose ethers, cellulose esters, polyvinyl alcohol, alginic acid esters, or other polymers having a colloidal or pseudocolloidal character.

Because of the tremendous variety of enzymes, for most material which is detrimental to dentures, appropriate enzymatic activity can be found for cleansing or supporting the cleansing procedure of the denture. It is the diversity of enzymes of animal origin and/or plant origin and/or microbial origin, allowing their use under various ionic strength and pH levels, that make them a very specific tool in removing plaque and/or stains and/or preventing further accumulation thereof. With regard to the chemical nature of many material which are detrimental to dentures, acidic proteases, alkali proteases and amylases, such as, for example, alpha-amylases, have been proven to be especially advantageous.

The use of an enzyme produced by genetic engineering techniques may be beneficial from a financial point of view, as said techniques allow a cheap production of the desired enzymatic activity to be added to the composition.

The wording "enzymes produced by genetic engineering techniques" is intended to include also those enzymes that have been modified by such techniques to make them more resistant to the chemical composition to which they are to be exposed. For example, other enzymatic activities contained in the inventive composition may attack the protein chain of the added enzymatic activity, thus limiting its efficacy. On the other hand, by introducing cleavage sites for which other added enzymatic activities are specific, a stringent control of the enzymatic activities is possible.

Alternatively, an enzyme isolated from biological material originally producing said enzymatic activity might also be of advantage, especially with regard to the fact that no cloning or the like is necessary and an abundant source for the respective enzymatic activity allows easy access to said enzymatic activity. Furthermore, such enzyme preparations gained from biological material may comprise a mixture of enzymatic activities that act in a synergistic manner, thus improving the overall denture cleansing and sanitizing process.

The synergistic effect and the stringent control of the enzymatic activities may especially result from a mixture of at least two enzymatic activities being incorporated in one single type of particles, with the particles being dispersed in the matrix and being visible discrete entities.

It is not necessary that the enzyme preparation used in the inventive composition has been isolated to homogeneity, although such an enzyme preparation might also advantageously be used with regard to avoidance of allergic reactions and to quality control. Enzyme preparations comprising further proteinaceous material are also useful because a cheaper production of the enzyme preparation is possible, as it is less time-consuming and additionally any loss of enzyme activity resulting from further purification steps is avoided. In addition, the further proteinaceous material can act as a filler to stabilize the particles containing at least one plaque and/or stain removing and/or preventing enzyme, so as to control the release of said particles from the water-soluble or water-dispersable matrix in which they are dispersed and/or of the enzyme from said particles themselves.

The enzyme exercises its beneficial effect in the denture cleansing procedure under circumstances that are most appropriate for its action, for example an alkali protease is released under an alkaline pH to, for example, remove food debris attached to the teeth.

The inorganic persalt bleaching agent(s) present in the composition of the invention further supports the denture cleansing efficacy of the inventive composition, as inorganic persalt bleaching agents have proven positive effects in denture cleansing.

A composition for use in denture cleansing further comprising percarbonate is especially advantageous with regard to the environmental performance of such a composition as, for example, percarbonate does not cause any problems in waste water treatment systems or in mineralization. Thus the addition of percarbonate as an inorganic persalt bleaching agent in the inventive composition, at least partially replacing other inorganic persalt bleaching agents which are more difficult to handle both from the point of view of manufacturing as well as from the point of view of environmental performance, improves the overall ecological balance of such a product and, in turn, improves its acceptance by customers.

Furthermore, the use of percarbonate as an inorganic persalt bleaching agent confers better solubility to the inventive composition.

The beneficial effects resulting from the addition of percarbonate as an inorganic persalt bleaching agent in said composition are furthermore increased when the content of percarbonate is high or when percarbonate is the only inorganic persalt bleaching agent in a composition according to the present invention.

The use of the or each or any combination of the inorganic persalt bleaching agent(s) as a particulate fraction or as visible discrete entities, or its use as part of any other particulate fraction or visible discrete entities of the composition, is advantageous for the same reasons as outlined above for the enzyme containing particles.

Embodiments of the present invention further comprising at least one organic peroxyacid bleach precursor of which many are known in the state of the art, exhibit additionally improved cleansing performance. Said organic precursor can advantageously be used if forming part of the particles containing at least one plaque and/or stain removing and/or preventing enzyme and/or being present in the matrix or being used and/or being present as an additional particulate fraction or as visible discrete entities.The latter aspect is useful for the same reasons as outlined above.

It must be clear that, from the point of view of stability, certain combinations of compounds in a particle, particulate fraction or visible discrete entities of the inventive composition are more preferred than others. For example combining TAED as a representative organic peroxyacid bleach precursor with any enzyme in a particle can be realized without any problems. In contrast, it is sometimes necessary to enhance the stability of a combination of an inorganic persalt bleaching agent and an organic peroxyacid bleach precursor in a single particle or the combination of an inorganic persalt bleaching agent and an enzyme in a single particle. For example, suitable coatings are available or appropriate fillers can be used to create a particle not allowing any direct contact of said compounds with each other, i.e. a compartmentalized particle or entity, which nevertheless exhibits the above-mentioned benefits arising from particles, regardless of whether they are present as particulate fractions or visible discrete entities.

To which fraction of the composition the organic peroxyacid bleach precursor should be added is determined according to the chemical composition and the pH phases to be realized. The particles and/or particulate fraction and/or visible discrete entities of the inventive composition, whatever their content may be, further comprising an effervescence generator, are especially useful to achieve a good cleansing performance, as said generator makes sure that the particles or fractions or entities are moved relatively to the aqueous medium, thus avoiding any gradient formation and making sure that no problems arising from transport limitatons or the like reduce the efficacy of the cleansing process.

The incorporation of an effervescence generator refers to particles or the like containing enzyme as well as to those containing percarbonate or peroxyacid bleach precursors or those containing at least two of said compounds. Besides this physical effect, the chemical effects arising from the effervescence generators are beneficial as they provide at least a micro-environment that is suitable for the optimum action of the compounds they are associated with. This includes the presence of oxygen for further attacking, for example, the food debris already attacked by the enzyme, or the other way round, preparing the food debris to be attacked by the enzymatic activity, as well as the generation of carbon dioxide being, for example, responsible for an effective dissolution of the composition and mechanical cleansing effects, thus further increasing the overall cleansing efficacy.

Effervescence generators which are effective under alkaline pH conditions include persalts such as alkali and alkaline earth metal peroxyborates as well as sodium perborate anhydrous, persulphates, percarbonates, perphosphates and mixtures of two or more thereof.

Effervescence generators which are effective under acid or neutral pH conditions include a combination of at least one alkali metal carbonate or bicarbonate, such as sodium bicarbonate, sodium carbonate, sodium sesquicarbonate, potassium carbonate, potassium bicarbonate or mixtures of two or more thereof in admixture with at least one non-toxic, physiologically acceptable organic acid, such as tartaric, fumaric, citric, malic, maleic, gluconic, succinic, salicylic, adipic or sulphamic acid, sodium fumarate, sodium or potassium acid phosphates, betaine hydrochlorides or mixtures of two ore more thereof.

The incorporation of a water-soluble or water-dispersable matrix comprising a solid base material is especially advantageous if the solid base material releases carbon dioxide and/or oxygen with effervescence, thus further improving the mixing within the aqueous medium, and in turn overcoming transport limitations and therefore increasing cleansing efficacy.

An inventive composition which is characterized by the inorganic persalt bleaching agent being selected from the group comprising alkali metal perborate and/or alkaline earth metal perborate and/or percarbonate, and the solid base material comprising at least one component phase having an acid or neutral pH in aqueous medium, at least one phase having incorporated therein at least a portion of the alkali metal perborate and/or the alkaline earth metal perborate and/or the percarbonate, allows excellent anti-plaque and anti-bacterial performance. Under these conditions, the enzyme containing particles exercise their beneficial effects or are excluded from participating in the respective cleansing step or phase characterized by an acid or neutral pH by appropriate measures such as coating or addition of fillers.

To further improve the overall cleansing performance of the inventive composition, the organic peroxyacid bleach precursor containing particles, particulate fraction or visible discrete entities, possibly containing other compounds such as enzymes or percarbonate, can have an alkaline pH in aqueous medium. Because of this alkaline pH, a second phase of the cleansing process can be established which, as known from the state of the art, is especially advantageous after an acid pH cleansing step or phase. Furthermore, the alkaline pH can provide appropriate pH conditions for another enzymatic activity contained in the inventive composition as particles being visible discrete entities.

On the other hand, an inventive composition being characterized by the inorganic persalt bleaching agent being selected from the group comprising alkali metal perborate and/or alkaline earth metal perborate and/or percarbonate, and a solid base material comprising at least one component phase having an alkaline pH in aqueous medium, said phase having incorporated therein at least a portion of the alkali metal perborate and/or the alkaline earth metal perborate and/or the percarbonate, offers another advantage that is helpful in the design of an appropriate composition for use in denture cleansing, by providing a more rapid dissolution of the composition.

Further advantages as outlined for a composition comprising a solid base material with at least one component phase having an acid or neutral pH in aqueous medium are also given in cases where the composition comprises at least one component phase having an alkaline pH, and in turn also for a composition characterized by the organic peroxyacid bleach precursor containing particles, particulate fraction or visible discrete entities, as defined herein, having an acidic or neutral pH in aqueous medium. Additionally, the acidic or neutral pH is more appropriate with regard to solution clarity, which is an important factor because of aesthetic considerations which will be taken into account by the customer.

The advantages as outlined in the above paragraphs are also true for compositions which also contain flavouring agents, agents for plaque removal, tartar removal, plaque control, tartar control, surface modification, natural teeth preservation, saliva regulation, bad breath neutralization, freshness, an anti-soil/anti-bacterial agent or a mixture of two or more thereof. If at least one of said agents or any combination thereof is used and/or is present as an additional particulate fraction or visible discrete entities the advantages as set forth for other particulate fractions or visible discrete entities according to the present invention are given, too, including the situation that at least one of said agents or any combination of said agents forms part of any other particulate fraction of the composition and/or any visible discrete entities contained therein.

In a preferred embodiment of the invention, the composition comprises about 0.5 to 5 wt % enzyme, about 10 to 40 wt % inorganic persalt bleaching agent and about 55 to 89.5 wt % matrix. Optionally, the composition comprises about 0.5 to 5 wt % enzyme, about 10 to 40 wt % inorganic persalt bleaching agent and also comprises an organic peroxyacid bleach precursor, preferably in an amount of about 0.5 to 5 wt %, and further additives such as flavouring agents and agents for plaque removal, with the balance being matrix.

Most preferably, the amount of inorganic persalt bleaching agent is about 15 to 35 wt %, and in particular about 23 wt % and the amount of enzyme is about 1.5 to 4.5 wt %.

In order that the invention may be more fully understood the following examples will now be described, by way of illustration only.

### Examples

Table 1 sets forth some compositions according to the present invention. All amounts given are percent by weight.

**Table 1**

| Ingredient | Ex. 1 | Ex. 2 | Ex. 3 | Ex. 4 | Ex. 5 | Ex. 6 | Ex. 7 | Ex. 8 |
|---|---|---|---|---|---|---|---|---|
| Potassium monopersulphate | 20,00 | 20,00 | 20,00 | 20,00 | 20,00 | 20,00 | 20,00 | 20,00 |
| Sodium perborate monohydrate | 3,0 | 3,0 | 3,0 | 3,0 | - | - | - | - |
| Sodium percarbonate | - | - | - | - | 3,0 | 3,0 | 3,0 | 3,0 |
| TAED | 1,00 | 1,00 | 1,00 | 1,00 | 1,00 | 1,00 | 1,00 | 1,00 |
| Sodium bicarbonate | 20,00 | 20,00 | 20,00 | 20,00 | 20,00 | 20,00 | 20,00 | 20,00 |
| Sodium carbonate | 10,00 | 10,00 | 10,00 | 10,00 | 10,00 | 10,00 | 10,00 | 10,00 |
| Citric acid anhydrous | 10,00 | 10,00 | 10,00 | 10,00 | 10,00 | 10,00 | 10,00 | 10,00 |
| Malic acid | 10,00 | 10,00 | 10,00 | 10,00 | 10,00 | 10,00 | 10,00 | 10,00 |
| Sulfamic acid | 3,00 | 3,00 | 3,00 | 3,00 | 3,00 | 3,00 | 3,00 | 3,00 |
| Sodium sulphate anhydrous | 11,99 | 11,99 | 11,99 | 8,99 | 11,99 | 11,99 | 11,99 | 8,99 |
| Sodium dodecyl benzene sulphonate | 0,50 | 0,50 | 0,50 | 0,50 | 0,50 | 0,50 | 0,50 | 0,50 |
| Flavour | 3,00 | 3,00 | 3,00 | 3,00 | 3,00 | 3,00 | 3,00 | 3,00 |
| PEG 4000 | 0,70 | 0,70 | 0,70 | 0,70 | 0,70 | 0,70 | 0,70 | 0,70 |
| PEG 6000 | 5,30 | 5,30 | 5,30 | 5,30 | 5,30 | 5,30 | 5,30 | 5,30 |
| Dyestuff | 0,01 | 0,01 | 0,01 | 0,01 | 0,01 | 0,01 | 0,01 | 0,01 |
| Coloured Protease like Novozym®243 | 1,50 | - | - | 1,50 | 1,50 | - | - | 1,50 |
| Coloured Amylase like Termamyl® | - | 1,50 | - | 1,50 | - | 1,50 | - | 1,50 |
| Coloured Lipase like Lipozyme™ | - | - | 1,50 | 1,50 | - | - | 1,50 | 1,50 |
| Total | 100 | 100 | 100 | 100 | 100 | 100 | 100 | 100 |
| Ex.: Example | | | | | | | | |

Among the above mentioned ingredients potassium monopersulphate, sodium perborate monohydrate, and sodium percarbonate are oxidizing agents supplying active oxygen to the solution.

TAED is an organic peroxyacid bleach precursor.

Sodium bicarbonate serves as a gaseous carbon dioxide supply, if supplied with organic acids. The gaseous carbon dioxide influences the dissolution characteristics of the composition and bubbles of carbon dioxide effect mechanical cleansing.

The organic acids are part of the effervescence system, serve as complexing agents and are involved in the regulation of the pH.

Sodium dodecyl benzene sulphonate is a detergent improving cleansing activity.

PEG 6000 and PEG 4000 are tabletting aids.

Sodium carbonate is part of the pH regulation system and a stability aid.

Sodium sulphate acts as a filler.

The enzyme containing particles have a diameter of 150 to 1000 µm. The enzyme particles comprise a coating consisting of sodium chloride, sodium sulphate or combinations thereof. Other compounds such as PEG might also be contained within said particles which serve as a binder. The enzyme containing particles are of red, green or blue color.

The features disclosed in the foregoing description and in the following claims may both separately and in any combination thereof, be material for realizing the invention in diverse forms thereof.

## Claims

1. A composition for use in denture cleansing comprising
a) particles containing at least one plaque and/or stain removing and/or preventing enzyme, and
b) a water-soluble or water-dispersable matrix, with the particles being dispersed in the matrix and being visible discrete entities and said composition further comprising at least one inorganic persalt bleaching agent.

2. A composition according to claim 1, wherein the enzyme is selected from the group comprising proteases, lipases and glycosidases, with the protease being preferably selected from the group comprising pepsin, papain, trypsin, acidic proteases or alkali proteases, and the glycosidase being preferably a glucanase and more preferably an endoglucanase or exoglucanase and most preferably an amylase.

3. A composition according to claim 1 or 2, wherein the inorganic persalt bleaching agent is an alkali metal persulphate, an alkali metal perborate, an alkali earth metal perborate, a percarbonate or a mixture of two or more thereof, and preferably the or each or any combination of the inorganic persalt bleaching agent(s) is used and/or is present as an additional particulate fraction and/or forms part of any other particulate fraction of the composition, and more preferably the or each or any combination of the inorganic persalt bleaching agent(s) being visible discrete entities and/or forming part of any other visible entities of the composition.

4. A composition according to any of claims 1 to 3, further comprising at least one organic peroxyacid bleach precursor.

5. A composition according to claim 4, wherein at least one organic peroxyacid bleach precursor forms part of the particles containing at least one plaque and/or stain removing and/or preventing enzyme or any other particles contained in the composition, and/or is present in the matrix, and/or is used and/or is present as an additional particulate fraction, preferably being visible discrete entities.

6. A composition according to claim 4 and 5, with the organic peroxyacid bleach precursor being selected from acylated polyalkyldiamines, especially tetraacetylethylenediamine, and carboxylic esters having the general formula AcL wherein Ac is the acyl moiety or an organic carboxylic acid comprising an optionally substituted, linear or branched C₆-C₂₀ alkyl or alkenyl moiety or a C₆-C₂₀ alkyl-substituted aryl moiety and L is a leaving group, the conjugate acid of which has a pKa in the range from 4 to 13.

7. A composition according to any of claims 1 to 6, with the particles and/or particulate fraction and/or visible discrete entities further comprising an effervescence generator.

8. A composition according to any of claims 1 to 7, wherein the water-soluble or water-dispersable matrix comprises a solid base material which preferably releases carbon dioxide and/or oxygen with effervescence.

9. A composition according to any of claims 1 to 8, further comprising a flavouring agent, an agent for plaque removal, tartar removal, plaque control, tartar control, surface modification, natural teeth preservation, saliva regulation, bad breath neutralization, freshness, an anti-soil/anti-bacterial agent or a mixture of two or more thereof, and preferably at least one of said agents or any combination of said agents is used and/or is present as an additional particulate fraction or visible discrete entities and/or forms part of any particulate fraction of the composition and/or any visible discrete entities contained therein.

10. A denture cleansing tablet containing a composition according to any of claims 1 to 9.
